# FASCICULE DE BREVET EUROPEEN

(11) **EP 2 790 605 B1**
(45) Date de publication et mention de la délivrance du brevet: **16.09.2020**
(21) Numéro de dépôt: 12812561.4
(22) Date de dépôt: 14.12.2012
(51) Int. Cl.: A61B 90/98, A61B 90/90, A61B 50/36

(54) **INSTRUMENT CHIRURGICAL COMPRENANT UN MOYEN DE DETECTION**
CHIRURGISCHES INSTRUMENT MIT EINER DETEKTIONSVORRICHTUNG
SURGICAL INSTRUMENT COMPRISING A DETECTION MEANS

(30) Priorité: 15.12.2011 FR 1161694
(43) Date de publication de la demande: 22.10.2014
(73) Titulaire: Khoury, Wassim, 75008 Paris (FR); Tagil Family Foundation, 9490 Vaduz (LI)
(72) Inventeur: KHOURY, Wassim, F-75008 Paris (FR)
(74) Mandataire: Gevers & Orès
(86) Numéro de dépôt international: PCT/EP2012/075667
(87) Numéro de publication internationale: WO 2013/087909

(56) Documents cités:
- EP-A2- 2 218 421
- WO-A1-96/22510
- WO-A1-2009/003231
- DE-A1-102010 022 086
- FR-A1- 2 937 860
- US-A1- 2006 065 739
- US-A1- 2007 125 392
- US-A1- 2011 036 738
- US-A1- 2011 174 877
- US-B1- 6 366 206

## Description

Le domaine de l'invention est celui des instruments chirurgicaux, notamment à usage unique, et en particulier des compresses.

Les compresses de gaze, d'utilisation courante en médecine et en particulier dans les spécialités de chirurgie telles que la chirurgie abdominale, gynécologique ou thoracique, servent notamment à éponger le sang lors d'une opération. Un problème majeur que rencontre les chirurgiens et les infirmiers lors de l'utilisation de telles compresses est le risque d'oubli d'une des compresses dans le corps du patient. Le risque est d'autant plus important qu'une fois imbibée de sang une compresse ressemble à une boule de graisse et peut ainsi être difficilement distinguable dans le corps d'un patient.

Afin de limiter ce risque, il est connu de compter le nombre de compresses avant et après l'opération afin de vérifier qu'aucune d'entres elles n'a été oubliée dans le corps du patient. Une telle opération de vérification engendre cependant une perte de temps importante pour les infirmiers qui doivent compter toutes les compresses avant et après l'opération, ainsi que pour le chirurgien qui doit attendre la validation de la vérification pour terminer son opération et recoudre le patient. En outre, le comptage des compresses à la fin de l'opération est une opération délicate posant des problèmes d'hygiène pour les praticiens, et en particulier les infirmiers du fait notamment de la manipulation des compresses imbibées de sang pouvant véhiculer de nombreuses maladies.

Dans le cas où une erreur est relevée entre le nombre de compresses avant et après l'opération, il est nécessaire d'effectuer un nouveau comptage et de vérifier en parallèle si la ou les compresses manquantes n'ont pas étés jetées par mégarde, par exemple lorsque le chirurgien retire ses gants d'opérations. Si le décompte n'est toujours pas équilibré, le chirurgien est alors amené à regarder attentivement dans le corps du patient à la recherche de la compresse qui comme on l'a dit précédemment peut aisément se confondre avec une boule de graisse. On comprend ainsi que l'utilisation des instruments de chirurgie génère des difficultés pratiques de gestion.

Afin de vérifier que le comptage a été bien réalisé ou dans le but de s'assurer que la ou les compresses manquantes ne sont pas dans le corps du patient, il est connu de disposer sur les compresses, au moment de leur fabrication, un fil de barite radio opaque afin de les rendre visibles aux rayons X. De telles compresses comprenant un fil de barite sont donc détectables grâce à des radios aux rayons X, normalement effectuées plusieurs heures après chaque opération.

Un premier inconvénient relatif à cette solution réside dans le fait que la résolution du système de radiographie utilisé doit être très élevée, de manière à détecter le fil de barite. Le principal inconvénient de cette méthode vient du fait qu'elle se déroule après l'opération, c'est-à-dire lorsque le patient a été recousu ou refermé. Dans ce cas, il est alors nécessaire de réopérer, avec tous les inconvénients d'une nouvelle intervention pour le patient, c'est-à-dire avec tous les risques que l'intervention chirurgicale peut engendrer pour le patient qui vient déjà de subir une opération parfois lourde, risques d'autant plus élevés si le patient est âgé ou présente des faiblesses de santé.

Dans le cas où la radio n'est pas de bonne qualité et qu'elle ne permet donc pas de détecter le fil de barite et la compresse, les conséquences pour le patient peuvent être encore plus grave, la compresse pouvant notamment provoquer une infection.

Que l'oubli soit diagnostiqué rapidement ou pas, cela peut avoir des conséquences dramatiques sur le patient comme vu précédemment mais aussi pour l'hôpital et le praticien responsable. Cela peut en effet engendrer de lourdes pertes financières pour l'hôpital du fait de la nouvelle opération et d'éventuelles poursuites judiciaires.

Dans ce domaine, il est connu des documenst WO 2009/003231, US 2006/065739 et US 2011/036738 différents dispositifs utilisant des puces RFID et des détecteurs associés. Le document DE 102010022086 divulgue pour sa part l'objet du préambule de la revendication 1.

L'invention vise ainsi à améliorer la situation.

Elle a ainsi vocation à être utilisée avec un instrument chirurgical comprenant un moyen de détection apte à réagir à au moins une sollicitation externe émise par un moyen générateur de la sollicitation.

On comprend ici que la sollicitation est externe au moyen de détection. La sollicitation est, par exemple, un signal, notamment un signal radio-fréquence. Suite à cette sollicitation externe, le moyen de détection réagit, par exemple en envoyant un signal, autrement appelé réaction, se rendant ainsi détectable par le moyen générateur.

Ainsi, grâce à l'invention, l'instrument chirurgical est détectable à tout moment, c'est-à-dire avant, pendant et après l'opération. L'instrument chirurgical est en outre détectable de manière instantanée, par exemple en quelques secondes ou en quelques minutes. Le praticien ou l'infirmier passe le moyen générateur proche du patient, le moyen de détection réagissant à une sollicitation émise par le moyen générateur. Autrement dit, si rien ne se passe, il n'y a pas de moyen de détection et donc pas d'instrument chirurgical. Au contraire, si le moyen de détection réagit, sa réaction est détectée par le moyen générateur et donne ainsi l'information qu'un instrument chirurgical se trouve dans la zone sondée, dite zone de contrôle.

Un tel procédé de détection est également transposable dans une situation où on détecte et on compte les instruments chirurgicaux préalablement à l'intervention, et où on sonde un contenant dans lequel les instruments chirurgicaux ont été rassemblés, postérieurement à l'intervention chirurgicale, en vue de fournir au praticien une information comparant la détection préalable à l'intervention et le sondage postérieur à l'intervention. Une telle détection et un tel sondage est réalisé par le moyen générateur.

Avantageusement, ledit instrument chirurgical est à usage unique. On entend par usage unique le fait que l'instrument chirurgical est destiné à être utilisé une seule fois, par exemple lors d'une seule intervention chirurgicale. L'instrument chirurgical à usage unique est stérile et jeté après l'intervention chirurgicale.

Selon un aspect de l'invention, le moyen de détection est apte à réagir à des radiofréquences. Avantageusement, le moyen de détection est une radio étiquette. La radio étiquette est agencée pour recevoir et répondre à la sollicitation, et ainsi rendre détectable l'instrument chirurgical sur lequel elle est installée. Le moyen générateur est, par exemple, un détecteur/émetteur de radiofréquences.

Dans une forme de réalisation de l'invention, le moyen de détection comprend un dispositif de solidarisation à l'instrument chirurgical. Le moyen de détection peut ainsi être solidarisé à l'instrument chirurgical, par exemple au moment de la fabrication de l'instrument chirurgical. Avantageusement, ledit dispositif de solidarisation comprend un matériau apte à être estampé. Le moyen de détection peut ainsi être estampé, par exemple, à chaud. De manière avantageuse, le matériau apte à être estampé est un polymère disposé au moins sur une périphérie du moyen de détection. Le matériau apte à être estampé est, par exemple, du polyuréthane ou du silicone.

De manière alternative, ledit dispositif de solidarisation comprend un matériau adhésif. Avantageusement, ledit dispositif de solidarisation comprend une bande adhésive. De manière avantageuse, le matériau adhésif et/ou la bande adhésive est appliqué sur une surface plane du moyen de détection. Le moyen de détection peut ainsi être collé sur l'instrument chirurgical.

Selon un aspect de l'invention, le moyen de détection est disposé au niveau d'une périphérie dudit outil chirurgical.

Selon un exemple de réalisation de l'invention, l'instrument chirurgical est une compresse, notamment une compresse de gaze. Le moyen de détection est disposé, par exemple, sur une périphérie de la compresse. De cette manière, lorsque la compresse est pliée, par exemple, pour être emballée ou pour s'adapter à la zone du patient sur laquelle elle va s'appliquer, le moyen de détection ne risque pas de perturber le pliage puisqu'il ne se trouvera pas dans une zone susceptible de gêner l'opération d'emballage ou l'utilisation de la compresse.

Avantageusement, le moyen de détection est solidarisé dans un pli de la compresse, notamment un pli situé sur une périphérie de la compresse. Le moyen de détection est ainsi entouré par la compresse. De cette manière, les faces extérieures, c'est-à-dire les faces planes, du moyen de détection sont protégées. On augmente en outre la tenue mécanique entre le moyen de détection et la compresse.

Décrit est aussi un procédé de fabrication d'un instrument chirurgical tel que décrit précédemment, dans lequel on solidarise le moyen de détection sur l'instrument chirurgical. Il est alors possible de solidariser le moyen sur l'instrument au moment de la fabrication de l'instrument, par exemple au moment du tissage de la compresse. Ce procédé de fabrication de l'instrument chirurgical est particulièrement adapté dans le cas où l'instrument chirurgical est à usage unique.

Selon un exemple de réalisation, on estampe le moyen de détection sur l'instrument chirurgical. On l'estampe, par exemple, à chaud.

Selon un exemple de réalisation, on colle le moyen de détection sur l'instrument chirurgical.

Dans ce cadre, l'invention concerne un moyen générateur d'une sollicitation d'une réaction provenant d'un moyen de détection d'instruments chirurgicaux, selon la revendication 1.

Ainsi, grâce à l'invention, lesdits instruments chirurgicaux sont identifiés au moment où ils passent devant le moyen générateur, fixé au moyen de réception. En positionnant le moyen générateur sur le moyen de réception et à la suite d'une intervention chirurgicale, tous les instruments chirurgicaux jetés dans le moyen de réception sont identifiés. Le caractère amovible du moyen générateur permet en outre d'identifier les instruments chirurgicaux à différents endroits et à différents moment de l'intervention chirurgicale comme expliqué ultérieurement.

Selon un exemple de réalisation de l'invention, ladite antenne est en forme d'anneau.

Selon un aspect de l'invention, ladite antenne est configurée pour être disposée sur ledit moyen de réception de sorte qu'elle définit un passage desdits instruments chirurgicaux dans ledit moyen de réception.

Selon un exemple de réalisation de l'invention, ladite antenne est configurée pour être disposée sur ledit moyen de réception de sorte qu'elle définit une entrée desdits instruments chirurgicaux dans ledit moyen de réception.

Ledit moyen générateur de la sollicitation est portatif. Il est ainsi possible de le déplacer dans des zones précises que l'on souhaite sonder, par exemple le champ opératoire ou un contenant rassemblant les instruments chirurgicaux. Avantageusement, ledit moyen générateur de la sollicitation présente une alimentation électrique autonome et/ou un écran de lecture des informations récoltées. Le praticien ou l'infirmier peut alors passer le moyen générateur autour du patient en étant libre de tout mouvement, c'est-à-dire sans gênes dues à la présence d'un fil d'alimentation.

De manière alternative (ne faisant pas partie de l'invention), ledit moyen générateur de la sollicitation est configuré pour pouvoir être fixé à un autre support. Ledit autre support est, par exemple, le sol d'une salle d'opération, une table opératoire et/ou une lampe d'éclairage de la zone d'intervention. Selon un exemple de réalisation, le moyen générateur est un portique déplaçable autour de la table d'opération, ou fixe à l'entrée du bloc opératoire.

Selon un exemple de réalisation, ledit moyen générateur de ladite sollicitation présente un dispositif d'identification du moyen de détection parmi une pluralité de moyen de détection. Le moyen de détection comprend une identification unique qui permet de l'identifier parmi d'autres moyens de détection, le moyen générateur étant apte à discriminer les instruments chirurgicaux portant des moyens de détection dont la réaction est différente les uns par rapport aux autres.

L'invention concerne aussi un ensemble comprenant un moyen générateur tel que décrit plus haut et un moyen de réception d'instruments chirurgicaux.

L'invention concerne également, un système comprenant un instrument chirurgical et un moyen générateur tels que décrits précédemment.

Comme déjà dit, ledit moyen générateur de la sollicitation pourra comprendre un moyen d'interprétation d'une réaction provenant du moyen de détection en réponse à la sollicitation. Le moyen de détection est ainsi apte à recevoir une sollicitation et à émettre une réaction en réponse à cette sollicitation. La réaction est, par exemple un signal. Ledit moyen générateur est ainsi apte à générer la sollicitation et à interpréter la réaction dudit moyen de détection en réponse à la sollicitation pour communiquer une information aux praticiens.

L'invention concerne aussi, un procédé de détection d'un instrument chirurgical par un moyen générateur, ledit moyen générateur et ledit instrument chirurgical formant un système tel que décrit précédemment, procédé dans lequel :
- on génère la sollicitation en direction d'une zone de contrôle,
- le ou les moyens de détection présents dans la zone de contrôle émettent chacun une réaction en réponse à la sollicitation,
- on détecte la ou les réactions du ou des moyens de détection.

Ce procédé de détection de l'instrument chirurgical est particulièrement adapté dans le cas où l'instrument chirurgical est à usage unique.

Dans une forme particulière du procédé de détection :
- on déplace le moyen générateur autour de la zone de contrôle.

Selon un aspect de l'invention, la zone de contrôle est une zone d'intervention chirurgicale du patient.

Il est ainsi possible de détecter le moyen de détection se trouvant dans la zone d'intervention et notamment de l'identifier de manière précise grâce au dispositif d'identification du moyen générateur.

De manière alternative :
- on identifie à l'aide du moyen générateur tous les instruments chirurgicaux porteurs d'un moyen de détection préalablement à l'intervention,
- on sonde tous les instruments chirurgicaux postérieurement à l'intervention chirurgicale, notamment en les disposant dans la zone de contrôle, à l'aide du moyen générateur,
- le moyen générateur compare l'identification préalable à l'intervention et le sondage postérieur à l'intervention et communique une information au praticien.

La zone de contrôle est par exemple le moyen de réception, notamment un contenant dans laquelle on jette tous les instruments chirurgicaux, une fois utilisé. Grâce au dispositif d'identification du moyen générateur, on identifie précisément chacun des moyen de détection présents dans la zone de contrôle et donc chacun des instruments chirurgicaux. On détecte ainsi la présence ou non de l'ensemble des instruments chirurgicaux utilisés pendant l'intervention chirurgicale.

Dans le cas où la zone de contrôle est le moyen de réception, le procédé de détection de l'invention prévoit une étape dans laquelle on dispose le moyen générateur sur le moyen de réception d'instruments chirurgicaux de sorte qu'ils forment l'ensemble selon l'une des revendications 4 ou 5.

Les instruments chirurgicaux peuvent être alors directement identifiés au moment où ils sont jetés dans le moyen de réception ou identifié au moment ou on positionne le moyen générateur sur le moyen de réception.

Selon un exemple de réalisation,
- l'étape d'identification préalable à l'intervention chirurgicale est effectuée en générant la sollicitation en direction d'au moins un emballage contenant tous les instruments chirurgicaux destinés à être utilisés lors de l'intervention chirurgicale.

Il est alors possible de comparer le nombre d'instrument chirurgical avant et après l'intervention et de détecter l'identification du ou des instruments chirurgicaux manquants.

Le caractère amovible de la fixation du moyen générateur au moyen de réception permet au moyen générateur d'effectuer cette comparaison. Il lui permet également d'identifier ailleurs que dans le moyen de réception un instrument chirurgical, éventuellement manquant lors de la comparaison effectuée avant et après l'intervention.

D'autres caractéristiques et avantages de l'invention apparaîtront encore mieux à la lecture de la description qui suit d'exemple de réalisation de l'instrument chirurgical de l'invention en référence au dessin annexé, sur lequel :
- la figure 1 est une vue schématique en plan d'un instrument chirurgical, notamment une compresse comprenant un moyen de détection apte à réagir à une sollicitation, et d'un moyen générateur de la sollicitation;
- la figure 2 est une vue détaillée en plan du moyen de détection de la figure 1 ;
- la figure 3 est une vue schématique en plan d'une variante de réalisation de la compresse représentée à la figure 1.
- la figure 4 est une vue schématique en perspective du moyen générateur et d'un moyen de réception formant un ensemble selon l'invention
- la figure 5 illustre une variant de la figure 4, lesdits moyens générateurs étant dans une position pliée où tels que destinés à être mis en place sur le moyen de réception et une position, dépliée, d'utilisation.

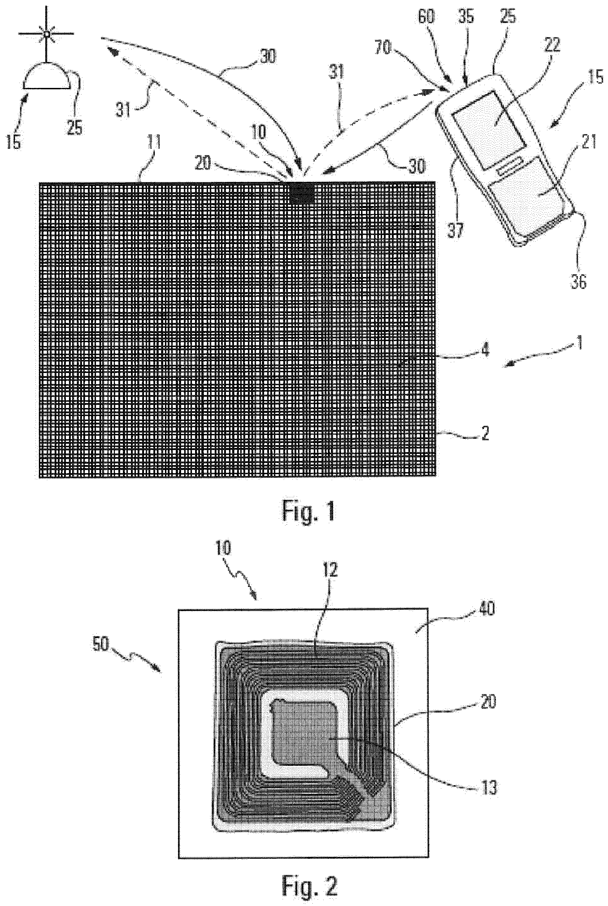

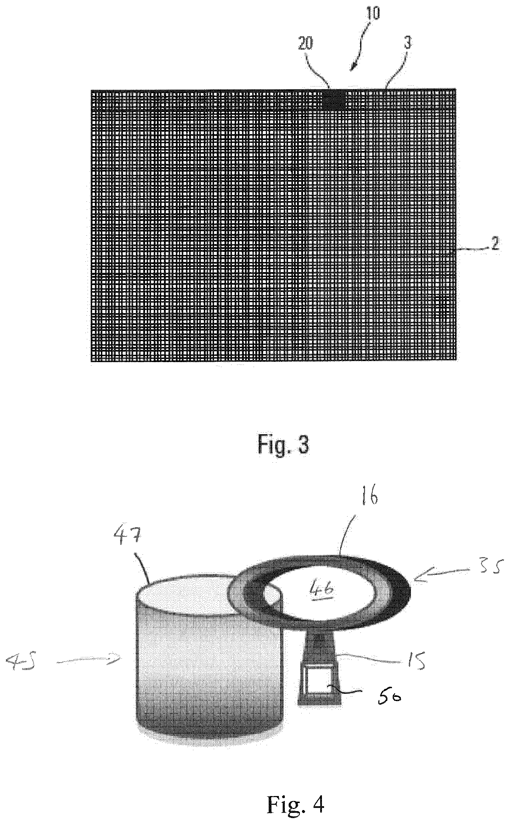

La figure 1 représente un instrument chirurgical, notamment à usage unique. Selon l'invention, l'instrument chirurgical 1 comprend un moyen de détection 10 apte à réagir à au moins une sollicitation 30 externe émise par un moyen générateur 15 de la sollicitation 30. L'exemple d'instrument chirurgical 1 représenté sur les figures 1 et 3 est ici une compresse 2, par exemple de gaze, et notamment stérile. Le moyen de détection 10 est configuré pour résister aux procédés de stérilisation connus à ce jour, par exemple par rayons gamma ou de préférence betta, ou encore par oxyde de méthylène. Ces procédés de stérilisation peuvent être réalisées à des températures inférieures à sensiblement 40°C.

Une telle compresse 2 comprend un tissage de fils 4, par exemple en fibre de gaze hydrophile en coton.

Le moyen de détection 10 illustré à la figure 1, et de manière plus détaillée à la figure 2, est une radio étiquette 20, ou autrement appelée marqueur. Elle comprend une antenne 12 associée à une puce électronique 13 qui lui permettent de réagir à une sollicitation externe 30, c'est-à-dire ici de recevoir la sollicitation 30, de l'analyser et d'y répondre. L'antenne 12 et la puce électronique 13 sont situées, notamment, au niveau d'une face avant de la radio étiquette 20.

La sollicitation 30 est émise par le moyen générateur 15, ici un lecteur 25 de radiofréquences. Il s'agit d'un émetteur et un récepteur de radiofréquences. La sollicitation 30 est, par exemple, une requête de radiofréquences émise depuis le lecteur 25 de radiofréquences. La radio étiquette 20 émet une réaction 31, ici un signal de réponse autrement appelé réaction, en réponse à la sollicitation externe 30 du lecteur 25 de radiofréquences. C'est notamment l'antenne 12 de la radio étiquette 20 qui émet la réaction 31. L'antenne 12 de la radio étiquette 20 est, par exemple, en cuivre. La détection de la radio étiquette 20 par le lecteur 25 de radio fréquence est ainsi de type radio-identification, plus souvent désignée par le sigle RFID (de l'anglais Radio Frequency Identification).

Les radios étiquettes 20 sont, par exemple, des dispositifs passifs, ne nécessitant aucune source d'énergie en dehors de celle fournie par le lecteur 25 de radiofréquences au moment de leur sollicitation. L'une des réactions est le renvoi d'une identification numérique, par exemple celle du standard EPC-96 qui utilise 96 bits.

La radio étiquette 20 de l'invention se situe, notamment, au niveau d'une périphérie 11 de l'instrument chirurgical 1, c'est-à-dire ici sur une périphérie de la compresse 2. On entend par périphérie 11 une zone située proche d'un bord de la compresse 2. Autrement dit, la radio étiquette 20 comprend une tranche longeant une partie du bord de la compresse 2, le reste de la radio étiquette 20 se trouvant sur la compresse 2.

Le lecteur 25 de radiofréquences est, par exemple, un dispositif actif, émetteur de la sollicitation 30 et récepteur de la réaction 31. Le lecteur 25 de radiofréquences active la radio étiquette 20 en lui fournissant ici, par l'intermédiaire de la sollicitation 30, l'énergie dont la radio étiquette 20 a besoin pour réagir. La fréquence utilisée est variable, selon le type d'application, les performances recherchées et la distance de détection ciblée. La fréquence d'utilisation peut également être fixe, notamment dans le cas où on utilise un lecteur 25 de radiofréquences portatif.

Le lecteur 25 de radiofréquences comprend pour cela un dispositif d'émission 60 de la sollicitation 30 lui permettant d'émettre la sollicitation 30. Il comprend en outre un dispositif de réception 70 de la réaction 31 lui permettant de réceptionner la réaction 31. Les dispositifs d'émission 60 de la sollicitation 30 et de réception 70 de la réaction 31 comprennent, notamment, une ou plusieurs antennes (référencée 16 sur la figure 4) par lesquelles la sollicitation 30 est émise et la réaction 31 réceptionnée.

Afin d'analyser la réaction 31 de la radio étiquette 20, le lecteur 25 de radiofréquences comprend un moyen d'interprétation 35. Le moyen d'interprétation 35 permet ainsi d'informer l'utilisateur, par l'intermédiaire d'un écran 22, de la présence ou non de la radio étiquette 20 dans la zone sondée, notamment dans la zone d'intervention, c'est-à-dire une zone située autour et au dessus du patient.

Le lecteur 25 de radiofréquences présente une alimentation électrique autonome 36, par exemple une batterie électrique. Il comprend en outre une zone de commande 21 depuis laquelle l'utilisateur du lecteur 25 de radiofréquences peut notamment commander l'émission de la sollicitation 30.

Le lecteur 25 de radiofréquences peut être portatif comme illustré en haut à droite sur la figure 1 ou fixé à un support comme représenté en haut à gauche de la figure 1. Dans le cas où il est portatif, il peut être déplacé simplement autour du patient ou proche de toute zone que l'on souhaite sonder, par exemple à une distance inférieure à 40 cm de la zone que l'on souhaite sonder, cette distance pouvant être réduite jusqu'au contact du lecteur 25 de radiofréquences avec le patient, en utilisant dans ce cas des pochettes stériles transparentes disposées entre le patient et le lecteur 25 de radiofréquences. Dans le cas où il est portatif, il peut également être fixé de manière amovible à un moyen de réception comme expliqué dans la suite.

Dans le cas où le lecteur 25 de radiofréquences est fixé à un support, le support peut être avantageusement la table opératoire ou encore une lampe d'éclairage de la zone d'intervention, de sorte que le lecteur 25 de radiofréquences se trouve proche du patient et de la zone d'intervention chirurgicale et qu'il peut émettre la sollicitation en direction de cette zone dans le but de détecter la radio étiquette 20. Dans le cas où le lecteur 25 de radiofréquences est fixé à un support, il doit être situé à une distance, par exemple égale à un mètre de la zone que l'on souhaite sonder.

Qu'il soit portatif ou fixé à un support, le lecteur 25 de radiofréquences présente un dispositif d'identification 37 de la radio étiquette 20. La réaction 31 de la radio étiquette 20 étant notamment une identification numérique qui utilise 96 bits, chaque radio étiquette 20 comprend sa propre identification numérique. Le dispositif d'identification 37 permet ainsi d'identifier précisément le numéro de la radio étiquette 20 et donc de la compresse 2 sur laquelle elle est située. Les compresses 2 étant rangées en séries, par exemple en séries de dix compresses, chaque série étant présente avant l'opération dans des emballages distincts, il est possible de savoir à quel emballage elle appartenait et donc de savoir à quel moment de l'opération elle a été utilisée en identifiant le numéro de la compresse 2 grâce à la radio étiquette 20. En cas d'oubli de la compresse 2, il est ainsi possible de limiter la recherche visuelle de la compresse 2 à la zone du corps du patient opérée au moment de l'utilisation de la compresse.

Selon une variante de réalisation, les radios étiquettes 20 sont, notamment, des dispositifs actifs. Elles comprennent dans ce cas leur propre source d'énergie. Elles n'ont donc pas besoin ici de recevoir de l'énergie du lecteur 25 de radiofréquences. Dans cette variante de réalisation, le lecteur 25 de radiofréquences ne fournit donc pas d'énergie à la radio étiquette 20.

La radio étiquette 20 résiste à l'humidité et jusqu'à des températures sensiblement égales à 200°C. Elle est notamment recouverte et protégée par deux films résistants à l'humidité et à des températures allant jusqu'à sensiblement 200°C. Les deux films sont, par exemple, en matériau polymère souple, notamment du polyuréthane. La radio étiquette 20 est extrêmement discrète par sa finesse, par exemple, quelques dixièmes de millimètres, sa taille réduite, notamment quelques millimètres, et sa masse négligeable. La radio étiquette 20 présente par exemple une longueur d'environ 30 mm et une largeur d'environ 10 mm. Son coût est minime. Elle comprend en outre des caractéristiques spécifiques la rendant souple et imperméable. C'est notamment grâce à son épaisseur réduite et les matériaux qui la constituent, en particulier son antenne en cuivre et ses films de protection en polymère souple, que la radio étiquette 20 est flexible. Cette caractéristique est importante du fait de la nécessité pour la compresse 2 d'être souple afin, notamment, d'être agencée de différentes manières pour s'adapter au mieux à la zone où elle va être appliquée. Une telle radio étiquette 20 est alors parfaitement adaptée pour être incorporée à la compresse 2 ou à un autre instrument chirurgical et être au contact, notamment, du sang ou de tous autres liquides présents dans le corps du patient, sans risquer d'être endommagée et sans risquer d'altérer significativement les caractéristiques intrinsèques de la compresse, notamment d'absorption.

La radio étiquette 20 comprend un dispositif de solidarisation 50 à la compresse 2. Ce dispositif de solidarisation 50 est situé, par exemple, sur une face arrière de la radio étiquette 20, c'est-à-dire la face opposé comportant la puce et l'antenne.

Le dispositif de solidarisation 50 peut également déborder d'une périphérie de la radio étiquette 20, en particulier sur toute la périphérie de la radio étiquette 20, c'est-à-dire tout autour de la radio étiquette 20. Le dispositif de solidarisation 50 forme alors une bande périphérique de la radio étiquette 20.

Le dispositif de solidarisation 50 comprend, ici, un matériau 40 apte à être estampé sur la compresse 2, un tel matériau étant par exemple thermo-comprimé sur la compresse. Le matériau 40 apte à être estampé sur la compresse 2 est, notamment, un polymère et par exemple un polyuréthane ou un silicone. Il est destiné à fondre à chaud, c'est-à-dire aux environs de 200°C, et à se solidifier à froid. Dans une première variante le matériau devient adhésif. Dans une autre variante, il se mêle aux fils 4 de manière à former un lien mécanique avec la compresse 2.

Dans une variante de réalisation de l'invention, le dispositif de solidarisation 50 comprend une matière adhésive, par exemple de la colle ou du silicone. On comprend par matière adhésive, une matière permettant de coller la radio étiquette 20 à la compresse 2. Elle est, par exemple appliquée contre une face plane de la radio étiquette 20, notamment une face arrière de la radio étiquette 20 destinée à être en contact avec la compresse 2, c'est-à-dire une face opposée à la face avant de la radio étiquette recevant l'antenne et la puce. Selon une autre variante de réalisation de l'invention, le dispositif de solidarisation 50 comprend une bande adhésive. On comprend par bande adhésive, une bande autocollante comprenant deux parois autocollantes, une première disposée contre la face arrière de la radio étiquette 20 et une deuxième permettant de coller la radio étiquette 20 à la compresse.

La figure 3 illustre un exemple de réalisation de l'invention selon lequel la radio étiquette 20 est située dans un pli de la compresse 2, en particulier un pli périphérique 3 de la compresse 2. On entend par pli périphérique 3 un pli situé au niveau d'une périphérie de la compresse 2. Le pli périphérique 3 est, par exemple, de la largeur de la radio étiquette 20 de sorte qu'il recouvre totalement la radio étiquette 20. La radio étiquette 20 est alors protégée de l'environnement extérieur puisqu'elle se trouve à l'intérieure de la compresse 2. Sa liaison mécanique à la compresse est également améliorée puisque le dispositif de solidarisation peut adhérer sur la compresse 2 et sur le pli périphérique 3 de la compresse.

La figure 4 illustre l'ensemble de l'invention comprenant le moyen générateur 15 et le moyen de réception 45 des instruments chirurgicaux. Le moyen de réception 45 est ici un bac ou corbeille dans lequel on jette les instruments chirurgicaux après les avoir utilisés dans une opération chirurgicale.

Le moyen de réception est, par exemple, en matière non métallique, notamment en carton ou en plastique. Il est ici de forme cylindrique et présente une un diamètre de 40 centimètres.

Selon l'invention, le moyen générateur 15 est agencé pour être fixé de manière amovible au moyen de réception. De cette manière, il peut au choix être déplacé ou fixé au moyen de réception.

Ledit moyen de réception 45 pourra présenter une armature de support d'un contenant jetable, destiné à recevoir lesdits instruments chirurgicaux, en particulier lesdites compresses, ledit moyen générateur 15 étant alors configuré pour être fixé, de façon amovible, à ladite armature.

L'antenne 16 du moyen générateur c omprend ici une ouverture 46 et se présente sous forme d'anneau. Le ou les instruments chirurgicaux sont alors destinés à passer à travers l'ouverture 46 lors de leur dépôt dans le moyen de réception 45 et peuvent ainsi être identifiés par le moyen générateur 15 grâce au moyen de détection 10.

L'antenne 16 du moyen générateur 15 est avantageusement disposée sur le moyen de réception 45 de sorte qu'elle définit un passage des instruments chirurgicaux dans le moyen de réception 45. On comprend ici que les instruments chirurgicaux sont destinés à passer dans une zone de sondage de l'antenne 16 permettant de les identifier lorsqu'ils sont jetés dans le moyen de réception 45.

En particulier, l'antenne 16 est disposée sur le moyen de réception 45 de sorte qu'elle définit une entrée des instruments chirurgicaux dans le moyen de réception 45. Elle est disposée alors sur un bord périphérique 47 du moyen de réception 45.

Ledit moyen générateur 15 pourra comporter un lecteur des informations récoltées. Ledit lecteur 50 est situé, par exemple, radialement.

Cela étant, ledit moyen générateur 15 est avantageusement configuré présenter une stabilité de positionnement une fois en place sur le moyen de réception 45. Il pourra en particulier présenter un centre d'inertie destiné à se trouver au niveau de la zone de passage pour les instruments chirurgicaux.

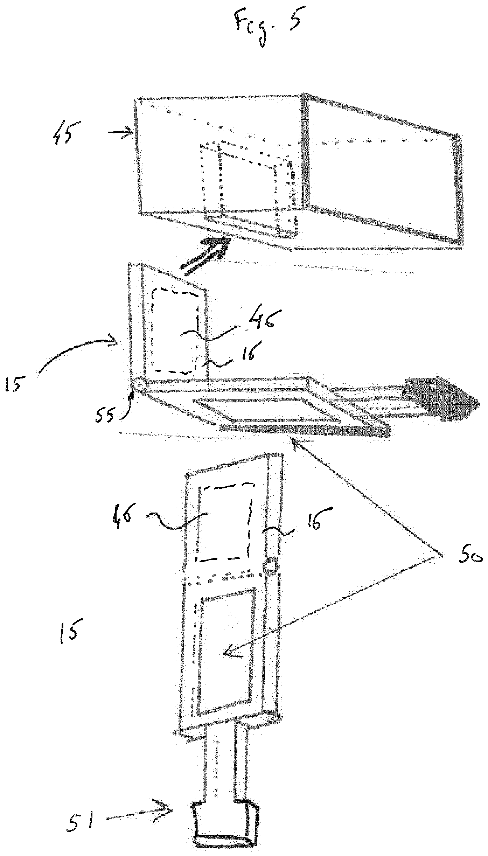

Comme illustré à la figure 5, il pourra comprendre deux parties amovibles l'une par rapport à l'autre, notamment par l'intermédiaire d'une charnière 55. La première desdites parties comprend, notamment, l'antenne 16 et/ou l'ouverture 46. Ladite seconde partie comprend, notamment, le lecteur 50 qui pourra ou non être prolongé d'une poignée 51.

Un tel générateur 15 pourra être configuré pour être positionné au niveau d'un moyen de réception 45 tel qu'une corbeille à tiroirs.

Décrit ici est aussi un procédé de fabrication de l'instrument chirurgical 1, dans lequel on solidarise le moyen de détection 10 sur l'instrument 1. On le solidarise, notamment, grâce au dispositif de solidarisation 50. On solidarise ici la radio étiquette 20 sur la compresse 2, en particulier sur la périphérie 11 et/ou dans le pli périphérique 3 de la compresse 2. Dans ce dernier cas, la radio étiquette 20 est alors solidarisée sur la compresse 2 avant ou après création du pli périphérique 3 qui vient recouvrir la radio étiquette 20.

Le procédé prévoit que l'on estampe le moyen de détection 10 sur la compresse 2 afin de la solidariser à cette dernière. L'estampage se fait par exemple à chaud. Le moyen de détection 10 comprend alors le matériau 40 apte à être estampé. L'estampage à lieu, notamment, sur la ligne de production du tissage de la compresse 2 de sorte que le procédé de fabrication de la compresse 2 n'est pas ou presque pas ralenti.

Le procédé prévoit une variante de réalisation selon laquelle on solidarise le moyen de détection 10 sur la compresse 2 par collage afin de le solidariser à cette dernière. Le moyen de détection 10 comprend alors, comme vu précédemment, la matière adhésive et/ou la bande autocollante. Dans ces cas également, le collage à lieu, notamment, sur la ligne de tissage de la compresse 2 de sorte que le procédé de fabrication de la compresse 2 n'est pas ou presque pas ralenti.

Selon un exemple de réalisation, la radio étiquette 20 est intégré à la compresse 2 pendant le tissage de la compresse 2. Les fils 4 sont, par exemple, cousus sur le dispositif de solidarisation 50, ce dernier pouvant être adhésif ou dépourvu de ce caractère adhésif.

A la suite de la solidarisation du moyen de détection 10 sur la compresse 2, la compresse 2 est découpée puis pliée avant d'être emballée.

L'invention concerne aussi un procédé de détection de l'instrument chirurgical 1 par le moyen générateur 15, dans lequel :
- on génère la sollicitation 30 en direction d'une zone de contrôle,
- le ou les moyens de détection 10 présent dans la zone de contrôle émettent chacun une réaction 31 en réponse à la sollicitation 30,
- on détecte la ou les réactions 31 du ou des moyens de détection 10.

Dans le cas où le moyen générateur 15 est portatif, comme illustré à la figure 1, on le déplace proche de la zone de contrôle que l'on souhaite sonder, c'est-à-dire proche de la zone où l'on souhaite identifier un ou plusieurs moyens de détection. Dans le cas où le moyen générateur 15 est solidarisé sur un support fixe, le moyen générateur 15 peut alors émettre la sollicitation 30 vers une zone précise, tout moyen de détection 10 passant par cette zone étant alors détecté. C'est par exemple le cas d'un moyen générateur solidarisé au sol à l'entrée du bloc opératoire.

La zone de contrôle est, par exemple, la zone d'intervention chirurgicale, c'est-à-dire une zone dans laquelle l'instrument chirurgical 1 est utilisé, notamment la zone dans laquelle le patient se trouve. On peut ainsi détecter directement la présence ou non du moyen de détection 10 sur le patient.

Selon une variante de l'invention,
- on positionne dans la zone de contrôle tous les instruments chirurgicaux 1 destinés à être utilisés au cours de d'une intervention chirurgicale à venir,
- le moyen générateur identifie ces instruments chirurgicaux,
- on sonde tous les instruments chirurgicaux présents dans la zone de contrôle postérieurement à l'intervention, le moyen générateur fournissant alors une information à l'attention du praticien pour lui indiquer sur le résultat de la comparaison est égal à zéro, ou non.

La zone de contrôle est par exemple un contenant dans lequel on jette tous les instruments chirurgicaux utilisés lors de l'intervention chirurgicale. En passant ensuite le moyen générateur 15 portatif proche du contenant, ou en faisant passer le contenant à proximité du moyen générateur fixe, le moyen générateur identifie précisément tous les moyens de détection présents et donc tous les instruments chirurgicaux présents afin de vérifier qu'aucun de ceux-ci ne manque par rapport à l'identification réalisée préalablement à l'intervention chirurgicale.

Afin de pouvoir identifier et comparer le nombre de moyens de détection 10 avant et après l'intervention chirurgicale, l'invention prévoit également qu'on génère la sollicitation 30 en direction d'au moins un emballage contenant une pluralité ou tous les instruments chirurgicaux 1 destinés à être utilisés lors de l'intervention chirurgicale. C'est par exemple le cas d'un emballage contenant dix compresses.

L'invention a été décrite grâce aux dessins sur lesquels un exemple de réalisation d'instrument chirurgical 1 est illustré, ici la compresse 2. Il est bien évident que l'invention s'applique à tous types d'instruments chirurgicaux, notamment des pinces en plastiques appelées « clips vasculaires », des ciseaux, ou encore des compresses de plus grandes dimensions appelées « champs abdominaux », avantageusement à usage unique. Il est alors possible de solidariser le moyen de détection dans l'instrument chirurgical selon un des exemples vus précédemment ou, par exemple, en noyant le moyen de détection dans un matériau plastique constitutif d'une partie de l'instrument chirurgical 1 selon l'invention.

De la même manière, l'invention a été décrite à l'aide des dessins en prenant pour exemple de moyen de détection 10, une radio étiquette 20, et pour exemple de moyen générateur un lecteur 25 de radiofréquences. Il est bien évident que l'invention concerne d'autres types de moyen de détection 10 apte à réagir à une sollicitation émise par un autre type de moyen générateur.

## Revendications

1. Moyen générateur (15) d'une sollicitation (30) d'une réaction provenant d'un moyen de détection d'instruments chirurgicaux, ledit moyen générateur comprenant un moyen d'interprétation (35) de ladite réaction (31), obtenue en réponse à ladite sollicitation (30), et une antenne (16) par laquelle la sollicitation (30) est émise et la réaction (31) réceptionnée, ledit moyen générateur (15) de la sollicitation (30) étant portatif de sorte qu'il est déplaçable dans des zones précises, **caractérisé par le fait que** ledit moyen générateur (15) est agencé pour être fixé de manière amovible à un moyen de réception (45) desdits instruments chirurgicaux (1), ladite antenne (16) d'émission présentant une ouverture (46) au travers de laquelle le ou les instruments chirurgicaux (1) sont destinés à passer lors de leur dépôt dans le moyen de réception (45).

2. Moyen générateur (15) selon la revendication 1, dans lequel l'antenne (16) est en forme d'anneau.

3. Ensemble comprenant un moyen générateur (15) selon l'une quelconque des revendications précédentes et un moyen de réception (45) d'instruments chirurgicaux (1).

4. Ensemble selon la revendication 3, dans lequel ladite antenne (16) est disposée sur ledit moyen de réception (45) de sorte qu'elle définit un passage desdits instruments chirurgicaux (1) dans ledit moyen de réception (45).

5. Ensemble selon la revendication 4, dans lequel ladite antenne (16) est disposée sur ledit moyen de réception (45) de sorte qu'elle définit une entrée desdits instruments chirurgicaux (1) dans ledit moyen de réception (45).

6. Système comprenant un instrument chirurgical (1) et un moyen générateur (15) de la sollicitation (30) selon l'une quelconque des revendications 1 ou 2, ledit instrument chirurgical (1) comprenant un moyen de détection (10) apte à réagir à au moins une sollicitation externe (30) émise par le moyen générateur (15), le moyen de détection (10) comprenant un dispositif de solidarisation (50) à l'instrument chirurgical (1) comprenant une bande adhésive, l'instrument chirurgical (1) étant une compresse (2), ledit moyen de détection (10) étant solidarisé dans un pli (3) de la compresse (2).

7. Système selon la revendication 6, dans lequel ledit moyen générateur (15) de la sollicitation (30) comprend un dispositif d'identification (37) du moyen de détection (10) parmi une pluralité de moyen de détection (10).

8. Procédé de détection d'au moins un instrument chirurgical (1) par un moyen générateur (15), ledit moyen générateur (15) et ledit instrument chirurgical (1) formant un système selon l'une quelconque des revendications 6 ou 7, procédé dans lequel :
- on génère la sollicitation (30) en direction d'une zone de contrôle,
- le ou les moyens de détection (10) présents dans la zone de contrôle émettent chacun une réaction (31) en réponse à la sollicitation (30),
- on détecte la ou les réactions (31) du ou des moyens de détection (10).

9. Procédé de détection selon la revendication 8 dans lequel :
- on identifie à l'aide du moyen générateur tous les instruments chirurgicaux (1) porteurs d'un moyen de détection (10) préalablement à une intervention chirurgicale,
- on sonde à l'aide du moyen générateur (15) tous les instruments chirurgicaux (1) postérieurement à l'intervention chirurgicale, notamment en les disposant dans la zone de contrôle,
- le moyen générateur (15) compare l'identification préalable à l'intervention et le sondage postérieur à l'intervention et communique une information.

10. Procédé de détection selon la revendication 8 ou 9 dans lequel la zone de contrôle est le moyen de réception (45) et dans lequel :
- on dispose le moyen générateur (15) sur le moyen de réception (45) d'instruments chirurgicaux (1) de sorte qu'ils forment l'ensemble selon l'une des revendications 3 ou 4.

## Patentansprüche

1. Erzeugungsmittel (15) einer Anfrage (30) für eine Reaktion, die von einem Detektionsmittel chirurgischer Instrumente stammt, wobei das Erzeugungsmittel ein Interpretationsmittel (35) der Reaktion (31), die als Antwort auf die Anfrage (30) erhalten wird, und eine Antenne (16), durch die die Anfrage (30) emittiert und die Reaktion (31) empfangen wird, umfasst, wobei das Erzeugungsmittel (15) der Anfrage (30) derart tragbar ist, dass es in bestimmten Gebieten beweglich ist, **gekennzeichnet durch** den Umstand, dass das Erzeugungsmittel (15) angeordnet ist, um auf entfernbare Art an einem Empfangsmittel (45) der chirurgischen Instrumente (1) fixiert zu werden, wobei die Emissionsantenne (16) eine Öffnung (46) aufweist, durch welche das oder die chirurgischen Instrumente (1) bestimmt sind, bei ihrer Hinterlegung in dem Aufnahmemittel (45) hindurchzugehen.

2. Erzeugungsmittel (15) nach Anspruch 1, wobei die Antenne (16) ringförmig ist.

3. Baugruppe, umfassend ein Erzeugungsmittel (15) nach einem der vorstehenden Ansprüche und ein Aufnahmemittel (45) chirurgischer Instrumente (1).

4. Baugruppe nach Anspruch 3, wobei die Antenne (16) auf dem Aufnahmemittel (45) derart angebracht ist, dass sie einen Durchgang der chirurgischen Instrumente (1) in dem Aufnahmemittel (45) definiert.

5. Baugruppe nach Anspruch 4, wobei die Antenne (16) auf dem Aufnahmemittel (45) derart angebracht ist, dass sie einen Eingang der chirurgischen Instrumente (1) in dem Aufnahmemittel (45) definiert.

6. System, umfassend ein chirurgisches Instrument (1) und ein Erzeugungsmittel (15) der Anfrage (30) nach einem der Ansprüche 1 oder 2, wobei das chirurgische Instrument (1) ein Detektionsmittel (10) umfasst, das in der Lage ist, auf mindestens eine durch das Erzeugungsmittel (15) emittierte externe Anfrage (30) zu reagieren, wobei das Detektionsmittel (10) eine Befestigungsvorrichtung (50) zu dem chirurgischen Instrument (1) umfasst, umfassend ein Klebeband, wobei das chirurgische Instrument (1) eine Kompresse (2) ist, wobei das Detektionsmittel (10) in einer Falte (3) der Kompresse (2) befestigt ist.

7. System nach Anspruch 6, wobei das Erzeugungsmittel (15) der Anfrage (30) eine Identifikationsvorrichtung (37) des Detektionsmittels (10) unter einer Vielzahl von Detektionsmittel (10) umfasst.

8. Detektionsverfahren mindestens eines chirurgischen Instruments (1) durch ein Erzeugungsmittel (15), wobei das Erzeugungsmittel (15) und das chirurgische Instrument (1) ein System nach einem der Ansprüche 6 oder 7 bilden, wobei in dem Verfahren:
- die Anfrage (30) in Richtung einer Kontrollzone erzeugt wird,
- das/die in der Kontrollzone vorhandene Detektionsmittel (10) jeweils eine Reaktion (31) als Antwort auf die Anfrage (30) emittieren,
- die Reaktion(en) (31) des oder der Detektionsmittel(s) (10) detektiert werden.

9. Detektionsverfahren nach Anspruch 8, wobei:
- mithilfe des Erzeugungsmittels alle chirurgischen Instrumente (1), die ein Detektionsmittel (10) tragen, vor einer chirurgischen Intervention identifiziert werden,
- mithilfe des Erzeugungsmittels (15) alle chirurgischen Instrumente (1) nach der chirurgischen Intervention sondiert werden, insbesondere, indem sie in der Kontrollzone arrangiert werden,
- das Erzeugungsmittel (15) die Identifikation vor der Intervention und die Sondierung nach der Intervention vergleicht und eine Information kommuniziert.

10. Detektionsverfahren nach Anspruch 8 oder 9, wobei die Kontrollzone das Aufnahmemittel (45) ist und wobei:
- das Erzeugungsmittel (15) auf dem Aufnahmemittel (45) chirurgischer Instrumente (1) derart angebracht wird, dass sie die Baugruppe nach einem der Ansprüche 3 oder 4 bilden.

## Claims

1. Means for generating (15) and urging (30) of a reaction coming from a surgical instrument detection means, said generating means comprising a means for interpreting (35) said reaction (31), obtained in response to said urging (30), and an antenna (16) by which the urging (30) is emitted and the reaction (31) received, said means for generating (15) the urging (30) being portable, such that it is movable in precise zones, **characterised by** the fact that said generating means (15) is arranged to be fixed removably to a means for receiving (45) said surgical instruments (1), said emission antenna (16) having an opening (46) through which the surgical instrument(s) (1) are intended to pass during their deposition in the receiving means (45).

2. Generating means (15) according to claim 1, wherein the antenna (16) is ring-shaped.

3. Assembly comprising a generating means (15) according to any one of the preceding claims and a means for receiving (45) surgical instruments (1).

4. Assembly according to claim 3, wherein said antenna (16) is arranged on said receiving means (45) such that it defines a passage of said surgical instruments (1) in said receiving means (45).

5. Assembly according to claim 4, wherein said antenna (16) is arranged on said receiving means (45) such that it defines an inlet for said surgical instruments (1) in said receiving means (45).

6. System comprising a surgical instrument (1) and a means for generating (15) the urging (30) according to any one of claims 1 or 2, said surgical instrument (1) comprising a detection means (10) capable of reacting to at least one external urging (30) emitted by the generating means (15), the detection means (10) comprising a device for securing (50) to the surgical instrument (1) comprising an adhesive strip, the surgical instrument (1) being a compress (2), said detection means (10) being secured in a fold (3) of the compress (2).

7. System according to claim 6, wherein said means for generating (15) the urging (30) comprises a device for identifying (37) the detection means (10) from among a plurality of detection means (10).

8. Method for detecting at least one surgical instrument (1) by a generating means (15), said generating means (15) and said surgical instrument (1) forming a system according to any one of claims 6 or 7, method wherein:
- the urging (30) is generated in the direction of a control zone,
- the detection mean(s) (10) present in the control zone each emit a reaction (31) in response to the urging (30),
- the reaction(s) (31) of the detection mean(s) (10) is/are detected.

9. Detection method according to claim 8, wherein:
- all the surgical instruments (1) which carry a detection means (10) are identified using the generating means prior to a surgical operation,
- all the surgical instruments (1) are sampled using the generating means (15) subsequent to the surgical operation, in particular by arranging them in the control zone,
- the generating means (15) compares the identification prior to the operation and the sampling subsequent to the operation and communicates an item of information.

10. Detection method according to claim 8 or 9, wherein the control means is the receiving means (45) and wherein:
- the generating means (15) is arranged on the means for receiving (45) surgical instruments (1) such that they form the assembly according to one of claims 3 or 4.
